(19)

**Europäisches Patentamt**
**European Patent Office**
**Office européen des brevets**

(11) **EP 4 752 891 A1**

(12) **EUROPEAN PATENT APPLICATION**

(43) Date of publication:
03.06.2026 Bulletin 2026/23

(21) Application number: 25202462.5

(22) Date of filing: 16.09.2025

(51) International Patent Classification (IPC):
*G16H 10/20* (2018.01)   *A61B 5/16* (2006.01)
*G06F 18/00* (2023.01)   *G06N 3/02* (2006.01)
*G06N 5/00* (2023.01)   *G06N 7/01* (2023.01)
*G16H 20/70* (2018.01)   *G16H 40/60* (2018.01)
*G16H 50/70* (2018.01)

(52) Cooperative Patent Classification (CPC):
G16H 20/70; A61B 5/165; G06F 18/00; G06N 3/02;
G06N 5/00; G06N 7/01; G06N 20/00; G16H 10/20;
G16H 40/60; G16H 50/70

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH LA MA MD TN**

(30) Priority: 29.11.2024 IN 202421093803

(71) Applicant: **Tata Consultancy Services Limited**
**Maharashtra (IN)**

(72) Inventors:
• **BHAVSAR, Karan Rajesh**
**400607 Mumbai, Maharashtra (IN)**

• **KIMBAHUNE, Sanjay Madhukar**
**400021 Mumbai, Maharashtra (IN)**
• **DESHPANDE, Parijat Dilip**
**411028 Pune, Maharashtra (IN)**
• **AWASTHI, Gauri**
**400021 Mumbai, Maharashtra (IN)**
• **SHINDE, Sujit Raghunath**
**400607 Mumbai, Maharashtra (IN)**
• **VISHWAKARMA, Harsh**
**453112 Indore, Madhya Pradesh (IN)**

(74) Representative: **Goddar, Heinz J.**
**Boehmert & Boehmert**
**Anwaltspartnerschaft mbB**
**Pettenkoferstrasse 22**
**80336 München (DE)**

(54) **METHODS AND SYSTEMS FOR AUTOMATED PERSONALIZED DESTRESSOR RECOMMENDATION BASED ON STRESSOR ESTIMATION**

(57)     The disclosure relates generally to methods and systems for automated personalized de-stressor recommendation based on stressor estimation. Conventional techniques for detecting the stress and the stressors are mostly manual and employ only some of the influencing parameters such as physiological parameters, behavioral parameters, and so on, thus leading to inaccurate detection of stressors. The present disclosure solves the technical problems in the art by detecting the stress using multi-model evaluation design including physiological changes, psychological changes, behavioral changes, environmental changes, and a bio-chemical indicator. Next, the stressors related to stress detection are identified using a large language model (LLM), which correlates with stress patterns to create a personalized model. Then, appropriate de-stressors are recommended using a recommendation lookup table.

FIG. 2

EP 4 752 891 A1

**Description**

CROSS-REFERENCE TO RELATED APPLICATIONS AND PRIORITY

The present application claims priority to Indian application no. 202421093803, filed on November 29, 2024.

TECHNICAL FIELD

The disclosure herein generally relates to stress management, and, more particularly, to methods and systems for automated personalized de-stressor recommendation based on stressor estimation.

BACKGROUND

Stress has become a serious concern for human life in the modern world, leading to various health related issues and lifestyle diseases. Stress may affect one's body, thoughts and feelings and one's behavior. There can be various reasons (referred to as stressors) that result in stress such as workload pressure (especially in information technology (IT) place), meeting targets, financial concerns, family concerns, lifestyle habits, and so on. While stress is a feeling of emotional strain, mental pressure and/or psychological pain, a stressor is a situation or event that causes stress. These events may be internal or external and vary according to person to person's response to that event.

Thus, knowing or detecting the stressors (stress symptoms) within a stipulated time can help manage the stress and accordingly improve the health of the person. However, conventional techniques for detecting stress and stressors are mostly manual which consumes a lot of time (for example, few months) and may not be accurate in stress detection due to various reasons that cause the stress and their interdependencies. Furthermore, the conventional techniques for automatic detection of stress and stressors are limited and mostly employ only some of the influencing parameters such as physiological parameters, behavioral parameters, and so on, thus leading to inaccurate detection of stressors. This will further affect the inaccurate detection of de-stressors that are used to relieve the stress of the person within the stipulated time.

SUMMARY

Embodiments of the present disclosure present technological improvements as solutions to one or more of the above-mentioned technical problems recognized by the inventors in conventional systems.

In an aspect, a processor-implemented method for automated personalized de-stressor recommendation based on stressor estimation is provided. The method including the steps of: receiving a value of each of one or more input parameters of a subject whose stressor is to be estimated, and a personalized de-stressor is to be recommended, wherein the one or more input parameters of the subject comprises one or more physiological parameters, one or more psychological parameters, one or more behavioral parameters, one or more environmental parameters, and a bio-chemical indicator; determining a stress level of the subject based on the value of each of the one or more input parameters using a predefined stress detection criterion, wherein the predefined stress detection criterion is defined with one or more boundary conditions associated with the one or more input parameters; validating the stress level of the subject using the value of the bio-chemical indicator and a ground truth bio-chemical indicator value, to obtain a validated stress level of the subject; estimating one or more stressors out of a plurality of predefined stressors, based on the validated stress level of the subject, using a pre-trained large language model (LLM) agent; and recommending a de-stressor for the subject against each of the one or more stressors estimated, using a pre-configured recommendation lookup table.

In another aspect, a system for automated personalized de-stressor recommendation based on stressor estimation is provided. The system includes: a memory storing instructions; one or more Input/Output (I/O) interfaces; and one or more hardware processors coupled to the memory via the one or more I/O interfaces, wherein the one or more hardware processors are configured by the instructions to: receive a value of each of one or more input parameters of a subject whose stressor is to be estimated, and a personalized de-stressor is to be recommended, wherein the one or more input parameters of the subject comprises one or more physiological parameters, one or more psychological parameters, one or more behavioral parameters, one or more environmental parameters, and a bio-chemical indicator; determine a stress level of the subject based on the value of each of the one or more input parameters using a predefined stress detection criterion, wherein the predefined stress detection criterion is defined with one or more boundary conditions associated with the one or more input parameters; validate the stress level of the subject using the value of the bio-chemical indicator and a ground truth bio-chemical indicator value, to obtain a validated stress level of the subject; estimate one or more stressors out of a plurality of predefined stressors, based on the validated stress level of the subject, using a pre-trained large language model (LLM) agent; and recommend a de-stressor for the subject against each of the one or more stressors estimated, using a pre-configured recommendation lookup table.

In yet another aspect, there are provided one or more non-transitory machine-readable information storage mediums comprising one or more instructions which when executed by one or more hardware processors cause: receiving a value of each of one or more input parameters of a subject whose stressor is to be estimated, and a personalized de-stressor is to be recommended, wherein the one or more input parameters of the subject comprises one or more physiological parameters, one or more psychological parameters, one or more behavioral parameters, one or more environmental parameters, and a bio-chemical indicator; determining a stress level of the subject based on the value of each of the one or more input parameters using a predefined stress detection criterion, wherein the predefined stress detection criterion is defined with one or more boundary conditions associated with the one or more input parameters; validating the stress level of the subject using the value of the bio-chemical indicator and a ground truth bio-chemical indicator value, to obtain a validated stress level of the subject; estimating one or more stressors out of a plurality of predefined stressors, based on the validated stress level of the subject, using a pre-trained large language model (LLM) agent; and recommending a de-stressor for the subject against each of the one or more stressors estimated, using a pre-configured recommendation lookup table.

In an embodiment, the one or more physiological parameters comprises a body temperature, a blood pressure, a blood sugar level, a fatigue tracking, a cardiac screening, and an activity tracking, the one or more psychological parameters comprises an emotion level, and an anxiety level, the one or more behavioral parameters comprises a sleep deprivation, an alcohol abuse detection, a smoking detection, and an activity pattern detection, and the one or more environmental parameters comprises one or more location related parameters of one or more environments, one or more temporal related parameters, and one or more digital related parameters, associated with the subject.

In an embodiment, the one or more stressors out of the plurality of predefined stressors are estimated based on the validated stress level of the subject, using the pre-trained large language model (LLM) agent, by: generating an initial dialogue context from the value of each of the one or more input parameters and the validated stress level of the subject, using the pre-trained LLM agent; initiating a multi-model dialogue with the subject using the initial dialogue context, to obtain an initial response from the subject; generating one or more successive dialogue contexts based on the initial response of the subject, using the pre-trained LLM agent; initiating the multi-model dialogue with the subject using the one or more successive dialogue contexts, to obtain one or more successive responses from the subject, until no successive response is received from the subject; and estimating the one or more stressors out of the plurality of predefined stressors, based on the initial response, the one or more successive responses received from the subject and value of each of the one or more input parameters, using a pre-trained stressor estimation model.

In an embodiment, the pre-configured recommendation lookup table is generated by: receiving one or more historical knowledge documents and one or more literature surveys, from a knowledge repository; identifying a plurality of stressors and a plurality of de-stressors, from the one or more historical knowledge documents and the one or more literature surveys using an information retrieval technique; creating a recommendation knowledge graph using the plurality of stressors and the plurality of de-stressors, using a Markov's Decision Process (MDP); and generating the pre-configured recommendation lookup table from the recommendation knowledge graph using a graph traversal technique.

In an embodiment, the bio-chemical indicator is a cortisol level.

BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1 is an exemplary block diagram of a system for automated personalized de-stressor recommendation based on stressor estimation, in accordance with some embodiments of the present disclosure.

FIG. 2 is an exemplary block diagram illustrating modules of the system of FIG. 1 for automated personalized de-stressor recommendation based on stressor estimation, in accordance with some embodiments of the present disclosure.

FIG. 3 illustrates an exemplary flow diagram of a processor-implemented method for automated personalized de-stressor recommendation based on stressor estimation, using the system of FIG. 1, in accordance with some embodiments of the present disclosure.

FIG. 4 is a flowchart showing the steps for estimating one or more stressors out of a plurality of predefined stressors, based on the validated stress level of the subject, using a pre-trained large language model (LLM) agent, in accordance with some embodiments of the present disclosure.

FIG. 5 is a flowchart showing the steps for generating a pre-configured recommendation lookup table, in accordance with some embodiments of the present disclosure.

FIG. 6 is an exemplary snapshot of a chart showing stress sensor signal patterns, in accordance with some embodiments of the present disclosure.

FIG. 7 is an exemplary chart for calculating weightages for de-stressor, in accordance with some embodiments of the present disclosure.
FIG. 8 shows an exemplary recommendation knowledge graph, in accordance with some embodiments of the present disclosure.

DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

The history of human evolution may go as far back as 2 to 6 million years. Early humans have survival challenges much like any other animal. These early humans, much before civilization began, had to have survived natural calamities, and would have had sudden encounters with wild animals. Thus, losing in such a survival challenge would literally mean losing their life. Therefore, their fears were real, amounting to them surviving or not surviving. Much like the animals, to these life-threatening fears, their response mechanism was a fight or flight response. This conditioned these humans to develop their sensory mechanism to sense and detect dangers, and to respond to such sudden threats. In such sudden encounters they needed a prompt burst of energy and strength either to fight or flee. Also, much like the fear response, again learning from animals, to hunt for food, they had to develop aggression and rage. Eventually, through the evolutionary period leading to civilization and agriculture, these fears (stressors), and their responses (stress response), unlike the animals, led to humans developing stress coping mechanisms.

While stress is a feeling of emotional strain, mental pressure and psychological pain, a stressor is a situation or event that causes to feel stressed. These events can be internal or external and vary according to each individual response to that event. Researchers have established a potential correlation between stress and a hypothalamic-pituitary-adrenal (HPA) axis. The HPA axis is a communication system between three endocrine organs, namely, the hypothalamus, the pituitary gland and the adrenal glands. The HPA axis regulates the hormones and body's stress reaction. The alignment of HPA axis is crucial for the body's stress management as it is directly in response to nervous system stimulation.

For example, the HPA axis releases hormones such as cortisol in response to stress. The adrenal medulla also releases adrenaline hormone under stress situations. The HPA axis produces a chain reaction of hormones under stressful conditions that eventually trigger the release of cortisol. It's a cascade of triggering which starts from the autonomic nervous system that triggers the hypothalamus to release corticotrophin-releasing hormone (CRH). The CRH further triggers the anterior pituitary to release adrenocorticotropic hormone (ACTH). The ACTH subsequently triggers the adrenal glands (adrenal cortex) to release the cortisol hormone. However, once the stress response ends, the cortisol triggers the hypothalamus to stop making the CRH thus curtailing the response. Conventional studies suggest that experiencing frequent or intense stress and other issues can cause dysfunction with the HPA axis also known as an overactive HPA while an underactive HPA is referred to as HPA axis suppression.

Thus, intense and chronic stress that leads to the HPA axis dysfunction with increased cortisol levels are detrimental and lead to the dysfunction of the immune system with increased inflammation and autoimmune conditions. Elevated levels of inflammation in the body result in disorders such as diabetes, obesity, cancer, high blood pressure and anxiety disorders. Moreover, the HPA axis dysfunction also plays a role in memory loss and neurodegenerative disorders. While the dysfunction increases cortisol, the HPA axis suppression results in a reduced cortisol response. This further results in an impaired stress response which invariably affects the body's defense response and thus exposes the body to potential severe and life-threating infections.

Further, stress has become an integral part of the human experience especially in workplaces (such as IT workplace) where most demanding and challenging tasks with a time limit are to be handled. Coping with these workplace stressors can take a toll on one's body and mind and eventually disrupt the HPA axis, thus causing health issues. Having said that, a little stress is considered good also as it motivates and helps to adjust to our environment. However, if the stress goes unchecked and starts affecting the mental state of the person or causing disorders then it's a cause of a big worry. As explained above about the relevance and correlation of stress with the body's HPA axis, it is imperative that the axis is regularly kept under check to avoid any healthcare scare.

Theoretically, early humans in their evolutionary phase must have encountered fears occasionally - once in a week (hunt), once in a season (weather). The response mechanism of the HPA axis produces cortisol to regulate the stress. Also theoretically, this mechanism is tuned to this occasional frequency of stress management (producing a sudden burst of energy to fight or flee). Now with the human's stress's evolutionary context, cut to today's time the nature of stress continues to stem from fears, and response mechanism also continues to remain similar, however, the stressors have changed in kind and frequency.

Thus, the stressors have translated into new frequent fears like at work, at home or at school, fear of meeting new people, fear of missing timelines, fear of the unpredictable future, fear of failure, fear of competition, etc. The problem begins when the stressors of the modem day have changed, the response mechanism is still tuned to the pace of evolutionary phase. That is when the HPA axis throttles because it is constantly invoked and overused. As highlighted earlier, the HPA axis manages physiological parameters such as BP (Blood Pressure), Glucose, and Carbohydrate Metabolism. Excessive stress can significantly affect the management of these bodily parameters, and in the present day the effects of imbalance in these parameters are quite well-known to cause lifestyle diseases.

Conventional techniques for detecting stress and the stressors are mostly manual and may not be accurate in stress detection due to various reasons that cause the stress and their interdependencies. Furthermore, the conventional techniques for detecting stress and the stressors mostly employ only some of the influencing parameters such as physiological parameters, behavioral parameters, and so on, thus leading to inaccurate detection of stressors.

The present disclosure solves the technical problems in the art with the methods and systems for automated personalized de-stressor recommendation based on stressor estimation. According to the present disclosure, the methods and systems begin by detecting stress using multi-model evaluation design including physiological changes, psychological changes, behavioral changes, environmental changes, and a bio-chemical indicator. Next, the stressors related to stress detection are identified using a large language model (LLM), which correlates with stress patterns to create a personalized model. Then, appropriate de-stressors are recommended using a recommendation lookup table.

In the context of the present disclosure, the words such as 'human', 'person', 'subject' are interchangeably used which mean a living being who is having a stress or stress level to be detected.

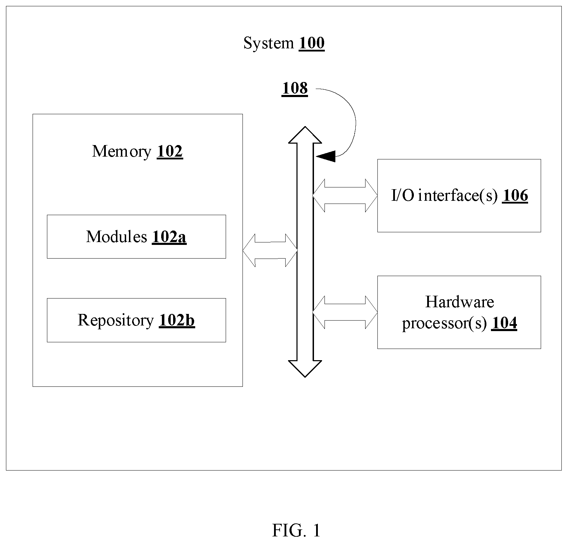

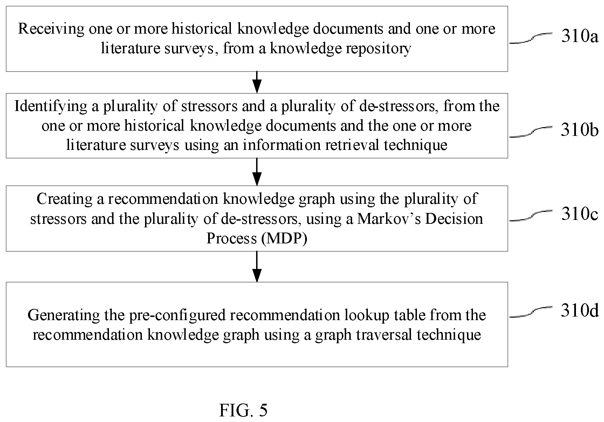

Referring now to the drawings, and more particularly to FIG. 1 through FIG. 8, where similar reference characters denote corresponding features consistently throughout the figures, there are shown preferred embodiments, and these embodiments are described in the context of the following exemplary system and/or method.

FIG. 1 is an exemplary block diagram of a system 100 for automated personalized de-stressor recommendation based on stressor estimation, in accordance with some embodiments of the present disclosure. In an embodiment, the system 100 includes or is otherwise in communication with one or more hardware processors 104, communication interface device(s) or input/output (I/O) interface(s) 106, and one or more data storage devices or memory 102 operatively coupled to the one or more hardware processors 104. The one or more hardware processors 104, the memory 102, and the I/O interface(s) 106 may be coupled to a system bus 108 or a similar mechanism.

The I/O interface(s) 106 may include a variety of software and hardware interfaces, for example, a web interface, a graphical user interface (GUI), and the like. The I/O interface(s) 106 may include a variety of software and hardware interfaces, for example, interfaces for peripheral device(s), such as a keyboard, a mouse, an external memory, a plurality of sensor devices, a printer and the like. Further, the I/O interface(s) 106 may enable the system 100 to communicate with other devices, such as web servers and external databases.

The I/O interface(s) 106 can facilitate multiple communications within a wide variety of networks and protocol types, including wired networks, for example, local area network (LAN), cable, etc., and wireless networks, such as Wireless LAN (WLAN), cellular, or satellite. For the purpose, the I/O interface(s) 106 may include one or more ports for connecting a number of computing systems with one another or to another server computer. Further, the I/O interface(s) 106 may include one or more ports for connecting a number of devices to one another or to another server.

The one or more hardware processors 104 may be implemented as one or more microprocessors, microcomputers, microcontrollers, digital signal processors, central processing units, state machines, logic circuitries, and/or any devices that manipulate signals based on operational instructions. Among other capabilities, the one or more hardware processors 104 are configured to fetch and execute computer-readable instructions stored in the memory 102. In the context of the present disclosure, the expressions 'processors' and 'hardware processors' may be used interchangeably. In an embodiment, the system 100 can be implemented in a variety of computing systems, such as laptop computers, portable computers, notebooks, hand-held devices, workstations, mainframe computers, servers, a network cloud and the like.

The memory 102 may include any computer-readable medium known in the art including, for example, volatile memory, such as static random access memory (SRAM) and dynamic random access memory (DRAM), and/or non-volatile memory, such as read only memory (ROM), erasable programmable ROM, flash memories, hard disks, optical disks, and magnetic tapes. In an embodiment, the memory 102 includes a plurality of modules 102a and a repository 102b for storing data processed, received, and generated by one or more of the plurality of modules 102a. The plurality of modules 102a may include routines, programs, objects, components, data structures, and so on, which perform particular tasks or implement particular abstract data types.

The plurality of modules 102a may include programs or computer-readable instructions or coded instructions that supplement applications or functions performed by the system 100. The plurality of modules 102a may also be used as, signal processor(s), state machine(s), logic circuitries, and/or any other device or component that manipulates signals based on operational instructions. Further, the plurality of modules 102a can be used by hardware, by computer-readable instructions executed by the one or more hardware processors 104, or by a combination thereof. In an embodiment, the plurality of modules 102a can include various sub-modules (not shown in FIG. 1). Further, the memory 102 may include information pertaining to input(s)/output(s) of each step performed by the processor(s) 104 of the system 100 and methods of the present disclosure.

The repository 102b may include a database or a data engine. Further, the repository 102b amongst other things, may serve as a database or includes a plurality of databases for storing the data that is processed, received, or generated as a result of the execution of the plurality of modules 102a. Although the repository 102b is shown internal to the system 100, it will be noted that, in alternate embodiments, the repository 102b can also be implemented external to the system 100, where the repository 102b may be stored within an external database (not shown in FIG. 1) communicatively coupled to the system 100. The data contained within such external database may be periodically updated. For example, data may be added into the external database and/or existing data may be modified and/or non-useful data may be deleted from the external database. In one example, the data may be stored in an external system, such as a Lightweight Directory Access Protocol (LDAP) directory and a Relational Database Management System (RDBMS). In another embodiment, the data stored in the repository 102b may be distributed between the system 100 and the external database.

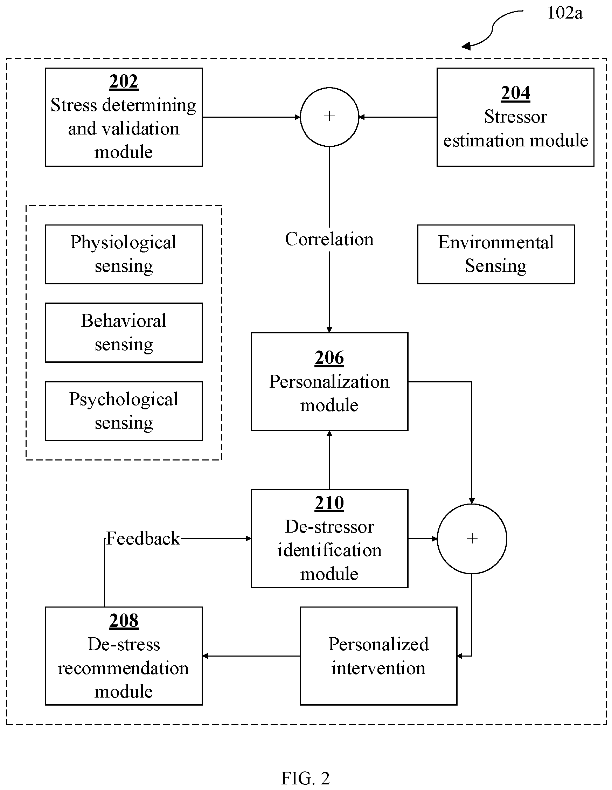

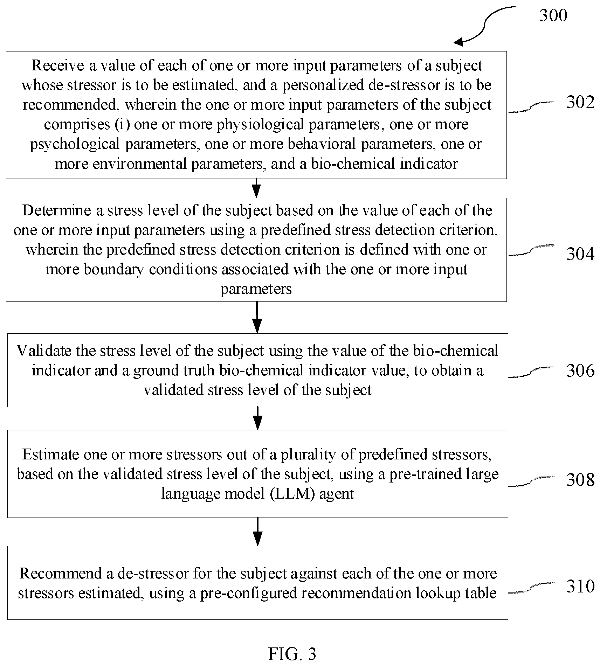

Referring collectively to FIG. 2 and FIG. 3, components and functionalities of the system 100 are described in accordance with an example embodiment of the present disclosure. For example, FIG. 2 is an exemplary block diagram illustrating modules 102a of the system 100 of FIG. 1 for automated personalized de-stressor recommendation based on stressor estimation, in accordance with some embodiments of the present disclosure In an embodiment, the plurality of modules 102a include a stress determining and validation module 202, a stressor estimation module 204, a personalization module 206, a de-stress recommendation module 208, and a de-stressor identification module 210.

For example, FIG. 3 illustrates an exemplary flow diagram of a processor-implemented method 300 for automated personalized de-stressor recommendation based on stressor estimation, using the system 100 of FIG. 1, in accordance with some embodiments of the present disclosure. Although the steps of the method 300 shown in FIG. 3 including process steps, method steps, techniques or the like may be described in a sequential order, such processes, methods, and techniques may be configured to work in alternate orders. In other words, any sequence or order of steps that may be described does not necessarily indicate a requirement that the steps be performed in that order. The steps of processes described herein may be performed in any practical order. Further, some steps may be performed simultaneously, or some steps may be performed alone or independently.

At step 302 of the method 300, the one or more hardware processors 104 of the system 100 are configured to receive a value of each of one or more input parameters of a subject whose stressor is to be estimated, and a personalized de-stressor is to be recommended. The one or more input parameters of the subject include: one or more physiological parameters, one or more psychological parameters, one or more behavioral parameters, one or more environmental parameters, and a bio-chemical indicator.

In accordance with the present disclosure, the one or more physiological parameters include but are not limited to a body temperature, a blood pressure, a blood sugar level, a fatigue tracking, a cardiac screening, and an activity tracking. In an embodiment, the fatigue tracking includes heart rate, eye tracking, and so on. In an embodiment, cardiac screening includes heart rate variability, breathing rate, SpO2, and so on. In an embodiment, the activity tracking includes but is not limited to walking/running, eating/drinking, and sitting/sleeping.

In accordance with the present disclosure, the one or more psychological parameters include but are not limited to an emotion level, and an anxiety level. The anxiety level is detected as a function inclusive of but not limited to sweat detection, heart rate detection, breathing rate detection, a multi-modal emotion detection such as audio, visual, textual content, or a combination thereof, and so on.

In accordance with the present disclosure, the one or more behavioral parameters include but are not limited to sleep deprivation, alcohol abuse detection, smoking detection, and activity pattern detection. In an embodiment, sleep deprivation includes but is not limited to attending meetings in late hours (during their night-time or rest time), responding to communications such as emails or Instant Messages (IMs) in late hours. In an embodiment, the alcohol abuse detection is performed through sensors and applications pre-installed in a GPS enabled wearable device and a mobile device, using a cumulative scoring technique.

In accordance with the present disclosure, the one or more environmental parameters include but are not limited to one or more location related parameters of one or more environments, one or more temporal related parameters, and one or more digital related parameters, associated with the subject. In an embodiment, the one or more location related parameters of one or more environments include but are not limited to home, office, in-transit, locational weather, traffic, noise, ambient odor, ambient temperature, ambient lighting, ambient air quality index (AQI), on office floor, meeting rooms, auditorium etc.

In an embodiment, the one or more temporal related parameters include but are not limited to time of day, day of week or month, calendar events, seasons like meetings, appraisals, corporate annual results, etc. In an embodiment, the one or more digital related parameters are time, duration of one or more digital devices such as laptop, mobile, tablet, App use (launch time / screen time), and so on.

In accordance with the present disclosure, the bio-chemical indicator is a cortisol level or any other indicator that is directly related to the stress measurement. In an embodiment, the cortisol level is measured either through invasive techniques or non-invasive techniques such as using a skin patch sensor based sweat analyzer to detect the amount cortisol.

In an embodiment, the value of each of one or more input parameters may be a number, a range, a textual information, or the like based on the nature of the one or more input parameters. For example, the value of the blood pressure is a number, the value of fatigue tracking is a time range or a textual input describing the fatigue related information, the activity pattern detection is a time range and a frequency interval of activities, and so on.

In an embodiment, the values of the one or more psychological parameters are obtained from the subject using a pre-configured questionnaire through either a manual or a digital survey. In an embodiment, the smoking detection of the subject is performed by measuring smoking episodes per day, sleeping hours per day, sleeping episodes per day, eating episodes per day, eating episodes per day, eating duration per episode, and so on.

At step 304 of the method 300, the one or more hardware processors 104 of the system 100 are configured to determine a stress level of the subject based on the value of each of the one or more input parameters received at step 302 of the method 300. In an embodiment, the stress determining and validation module 202 is configured to determine the stress level of the subject. In an embodiment, the stress level of the subject indicates whether the subject has stress or not. In another embodiment, the stress level of the subject indicates the amount of stress the subject has. The amount of stress may be represented on a scale 0 to 5, where 0 indicates no stress and 5 indicates maximum stress level.

In the present disclosure, a predefined stress detection criterion is employed to determine the stress level of the subject based on the value of each of the one or more input parameters. The predefined stress detection criterion is defined with one or more boundary conditions associated with the one or more input parameters considered at step 302 of the method 300.

In an embodiment, the values of each of one or more input parameters are modeled as a feature set for modelling the stress and the stress pattern. An exemplary feature set is modeled as below:

1. Given day is split into $x$ number of slots of size $ss$.
2. slot-index ($si$): Each slot is treated as a bucket of parameters mentioned below.

    a. An activity type ($at[si]$) is defined as: Running, Walking, Eating, Drinking, Driving (in commute), and special case for any uncategorized activity for any unknown state.
    b. An activity-intensity ($ai[si]$) is defined as a function of speed (min/km) and heart rate (bpm)
    c. The heart-rate ($hr[si]$) is defined during the timeslot.

3. Further a longitudinal data of these parameters such as below moving average of $n$ number of days, is calculated as:

    a. $aima_n[si]$ is a moving average of activity intensity for $n$ number of days for a specific slot-index [$si$].
    b. $hrma_n[si]$ is a moving average of heart rate for $n$ number of days for a specific slot-index [$si$]..
    c. $aisd_n[si]$ is a standard deviation on moving average data of $n$ number of days for activity intensity for a specific slot-index [$si$]..
    d. $hrsd_n[si]$ is a standard deviation on moving average data of $n$ number of days for heart rate for a specific slot-index [$si$]..
    e. $patma_n[si]$ is a primary activity type detected during a specific slot-index using histograms (count the number of activities of a particular type repeatedly performed in the specific slot index and choosing the one with highest count as primary activity). This helps formulate behavioral pattern of the subject. Example: Every morning at roughly 6 AM, the subject goes out for running, eats his lunch at around 1 PM, snacks at around 6 PM and dines at around 9 PM.

4. Consecutive slot-group-size ($sgs$) is considered as a parameter which is used to evaluate a group of consecutive slots to look at a larger time window.
5. The slot-size ($ss$), number of days ($n$) for evaluating moving average, and slot-group-size ($sgs$) are adjusted to fine-tune the computations. If any specific patterns are detected in slot-groups then these groups are recorded as episodes having respective unique episode_ids.
6. Beyond this each activity type may have sub parameters (features) such as:

    a. Smoking: smoking episodes per day ($avgSmokingEpisodesPerDay$)
    b. Sleeping: sleeping hours per day, sleeping episodes per day ($avgSleepingHoursPerDay$)
    c. Eating: eating episodes per day, eating duration per episode ($avgEatingEpisodesPerDay$)
    d. One or more location related parameters such as working beyond regular office hours. For example, $avgOfficehours$ and $augOverOfficeHours.$
    e. One or more digital parameters such as counting the number of events of checking and responding. For example, $workEmailsBeyondOfficeHours,$ $attendingMeetingsBeyondOfficeHours,$ and $schedulingMeetings$-

*BeyondOfficehours*

f. All such indicators are used to flag of behavioral changes (positive / negative).

7. Other features as per activity types are as below:

a. Identify the slot-groups with smoking activity for example. The activity types may vary (*pat*[*episode*] == *smoking*)

b. Register smoke detection values for the identified slot-group and compute an average smoke detection value for this particular slot-group (*cvSmokingAvg*[*episode*])

c. A moving average of smoke detection value over multiple smoking episodes (*cvSmokingMovingAvg*[*episode*]).

In an embodiment, the predefined stress detection criterion is formed using the above explained parameters for the defined events to detect the stress and the stress pattern:

1. The one or more events such as the ones shown below (but not limited to) are identified using an exemplary predefined stress detection criterion:

a. When a particular parameter is outside it's normal range in the exemplary predefined stress detection criterion:

$$(! \left( (aima_n[si] - aisd_n[si]) \leq ai[si] \leq (aima_n[si] + aisd_n[si]) \right) == true \, \| \, \&\&$$

$$! \left( (hrma_n[si] - hrsd_n[si]) \leq hr[si] \leq (hrma_n[si] + hrsd_n[si]) \right) == true \,)$$

b. When a parameter combination is in inverse relationship with each other: for example, the activity intensity is way lower than the regular range, but the heart rate is way higher than the regular range.

$$[ai[si] \leq (aima_n[si] - aisd_n[si]) \, ] \&\& [ \, hr[si] \geq (hrma_n[si] + hrsd_n[si])] == true$$

c. Behavioral change events

avgSmokingEpisodesPerDay

> movingAvgSmokingEpisodesPerDay

+ avgSmokingEpisodesSD avgSleepingHoursPerDay

< movingAvgSleepingHoursPerDay - avgSleepingHourSD

2. The slot-indexes for which one or more of the above exemplary predefined stress detection criterion (conditions) meet, then those slot-indexes are marked as probable-stress-patterns. An exemplary probable-stress-pattern is shown below:

```
"probable-stress-pattern" :
        {
                "slot-index" : 4,
                "slot-timestamp": "2024-09-08T18:23:41-12:00",
                "ai": 0.91234554,
                "ai_ma": 0.85234214,
                "ai_sd": 0.24062022
                "pat": "uncategorized",
```

```
         "hr": 145,
         "location": "19.214329602085254, 72.97309107038605",
       "address_components" : [
       {
         "long_name" : "ABC Rd No 2",
         "short_name" : "ABC Rd No 2",
         "types" : [ "street_name" ]
       },
       {
         "long_name" : "XYZ city",
         "short_name" : " XY city ",
         "types" : [ "city", "district" ]
       },
       {
         "long_name" : " PQR city ",
         "short_name" : "PQ city ",
         "types" : [ "corporate", "corporate_name" ]
       },
       {
         "long_name" : "AB Nagar",
         "short_name" : "PQX",
         "types" : [ "sublocality" ]
       },
       {
         "long_name" : "Country",
         "short_name" : "YZA",
         "types" : [ "country" ]
       },
       {
         "long_name" : "400601",
         "short_name" : "400601",
         "types" : [ "postal_code" ]
       }
     ],
       "reasons": [
                 "Detected uncategorized activity",
                 "Activity intensity higher than average",
                 "Activity intensity outside of standard deviations
         range"
                 ]
       }
```

3. Then slot-groups are identified for probable-stress-patterns and if the count is greater than or equal to a set threshold, then these slot-groups collectively are probable-stress-episodes.

```
{
"$probable-stress-episodes": [
       {
             "slot-group" : [4,5,6,7,8,9],
             "slot-timestamp": "2024-09-08T23:23:41-12:00",
             "ai": 0.912345545,
             "pat": "uncategorized",
             "hr": 145,
             "location": "19.214329602085254, 72.97309107038605",
             "reasons": [
                     "Detected uncategorized physical activity",
                     "Physical activity intensity higher than average",
                     "Physical activity intensity outside of standard
                     deviations range",
                     "Physical activity episode lasted longer than usual",
                     "Physical intense activity episode in late evening"
                         ]
                         ]
       }
```

4. In the presence of probable-stress-episode on a particular day, the subject is detected as 'stressed'.

At step 306 of the method 300, the one or more hardware processors 104 of the system 100 are configured to validate the stress level of the subject determined at step 304 of the method 300. In an embodiment, the stress determining and validation module 202 is configured to validate the stress level of the subject determined at step 302 of the method 300. In accordance with the present disclosure, the value of the bio-chemical indicator received at step 302 of the method 300 is used and compared against a ground truth bio-chemical indicator value, to validate the stress level determined at step 304 of the method 300. The stress level validated at this step is termed as a validated stress level of the subject. The exemplary ground truth bio-chemical indicator value ranges from 8.16 ng/mL to 141.7 ng/mL if the bio-chemical indicator is the cortisol level which is measured through sweat.

The validated stress level of the subject is the confirmed stress level (or status) whether the subject has stress or not. In an embodiment, if the stress level of the subject determined at step 304 indicates 'having stress' and the value of the bio-chemical indicator received at step 302 is greater than or equal to the ground truth bio-chemical indicator value, then the validated stress level of the subject indicates 'having stress', otherwise the validated stress level of the subject indicates 'not having stress' and subsequent steps are terminated incase the subject is not having the stress.

In another embodiment, the stress level detected at step 304 of the method 300 is validated using one or more criteria related to the bio-chemical indicator. An exemplary more criteria related to the bio-chemical indicator is as follows:

$$! \big( (cvma_n[si] - cvsd_n[si]) \leq cv[si] \leq (cvma_n[si] + cvsd_n[si]) \big) == true$$

wherein, $cvma_n[si]$ is a moving average of cortisol-value for $n$ number of days for a specific slot-index $[si]$, $cvsd_n[si]$ is a standard deviation on moving average data of $n$ number of days for cortisol-value for a specific slot-index $[si]$, the cortisol-value ($cv[si]$) is defined through science and literature. However, there may be momentary ups and downs based on specific events during the day. Also, the levels may vary for individuals. Hence, measuring cortisol-value for each slot-index would give the circadian rhythm pattern for the subject under evaluation.

Detection of chronic stress, which is a pre-curser to a whole range of diseases such as heart disease, cancer, lung ailments, accidents, cirrhosis of the liver, and suicide are enabled via monitoring cortisol. Such a biosensor will offer significant insights into the health and wellbeing of the person. The data can then be analyzed with respect to the various features it offers such as AUC, slope, etc. to achieve a correlation between various medical conditions and cortisol levels. Not only will this biosensor be beneficial to various subjects (patients) with chronic stress-related ailments but also elite athletes during their peak training sessions.

The feedback offered from continuous monitoring also allows for dosing appropriate quantities of supplements/medicines to offer a cure for their condition. A wearable sensor capable of collecting readings for the cortisol levels may benefit by offering an estimate on their chronic levels of stress and their response to treatments including medication and psychosomatic therapies. A typical therapy cycle continues for approximately 7 days for appreciable results and therefore a continuous monitoring sensor is the need of the hour.

The levels of the cortisol are typically measured during intervals listed below and range between

- 06.00 - 10.00 A.M.: 6.02 - 18.4 $\mu$g/dL and
- 04.00 - 08.00 P.M.: 2.68 - 10.5 $\mu$g/dL.

It is traditionally measured via ELISA cortisol antigen via ElectroChemiLuminescence Immunoassay. This, however, involves invasive and frequent sample collection, and though well established the therapeutic advantages remain limited. Cortisol is tested to determine cortisol blood level, which can be measured in three ways -- through blood, saliva (90% accurate), or urine. Since the levels of the cortisol vary significantly during the day, frequent samples are necessary e.g. urine samples are required every time.

At step 308 of the method 300, the one or more hardware processors 104 of the system 100 are configured to estimate one or more stressors out of a plurality of predefined stressors, based on the validated stress level of the subject obtained at step 306 of the method 300. If the validated stress level confirms that the subject is having stress then, the one or more stressors that cause the stress of the subject are estimated. In an embodiment, the stressor estimation module 204 is configured to estimate the one or more stressors out of the plurality of predefined stressors, based on the validated stress level of the subject obtained at step 306 of the method 300.

In the present disclosure, a pre-trained large language model (LLM) agent is employed to estimate the one or more stressors that cause the stress of the subject. In an embodiment, the pre-trained large language model (LLM) agent is obtained by either finetuning or configuring the conventional LLMs such as ChatGPT and LIAMA with one or more prompts suitable for this objective. FIG. 3 is a flowchart showing the steps for estimating one or more stressors out of a plurality of predefined stressors, based on the validated stress level of the subject, using a pre-trained large language model (LLM) agent, in accordance with some embodiments of the present disclosure. As shown in FIG. 3, estimating the one or more stressors is explained through steps 308a to 308e.

At step 308a, an initial dialogue context is generated from the value of each of the one or more input parameters received at step 302 of the method 300 and the validated stress level of the subject obtained at step 306 of the method 300. In the present disclosure, the pre-trained LLM agent is employed to generate the initial dialogue context from the value of each of the one or more input parameters if the subject is having the stress after the validation at step 306 of the method 300. The initial dialogue context indicates the abnormalities present in the subject based on the value of each of the one or more input parameters.

At step 308b, a multi-model dialogue is initiated with the subject using the initial dialogue context generated at step 308a, to obtain an initial response from the subject. The multi-model dialogue is initiated either with an audio conversation, a video conversation, or a textual conversation with the subject using the initial dialogue context. The multi-model dialogue is initiated to estimate probable stressors by having the dialogue with the subject and based on the response received from the subject.

At step 308c, one or more successive dialogue contexts are generated based on the initial response of the subject received at step 308b, using the pre-trained LLM agent. The one or more successive dialogue contexts are generated either one after another or simultaneously by having the continuous interaction with the subject based on the initial response and the subsequent responses.

At step 308d, the multi-model dialogue is initiated with the subject using the one or more successive dialogue contexts generated at step 308c, to obtain one or more successive responses from the subject. The multi-model dialogue is continued until no successive responses are received from the subject. The one or more successive responses are received from the subject either one after another or simultaneously based on the subsequent dialogue contexts and the subsequent responses.

At step 308e, the one or more stressors are estimated based on the initial response obtained at step 308b, the one or more successive responses received from the subject at step 308d, and value of each of the one or more input parameters received at step 302 of the method 300. A pre-trained stressor estimation model is employed to estimate the one or more stressors of the subject. The plurality of predefined stressors includes a list of stressor types identified in the art and the pre-trained stressor estimation model classifies the one or more stressors from this list. Table 1 shows a relationship table between exemplary stressors and the stress patterns.

Table 1

| stress-pattern | stress-labels | Stressors | Stressor-groups |
|---|---|---|---|
| Activity indicators, cortisol value indicators | Physical, emotional, fear | Emails | Emails, Meetings, Deadlines |

In an embodiment, the pre-trained stressor estimation model is obtained by training a machine learning (ML) model such as a random forest, support vector machine (SVM), or a deep learning (DL) model such as convolution neural networks (CNN), with suitable annotations comprising the stress patterns and the stressors.

For example, training the CNN model to obtain the pre-trained stressor estimation model is as follows: Firstly, the input parameters are summarized as stress patterns based on their values. For example, (i) Activity sensors report high activity, (ii) Cardiac sensors report abnormality, (iii) Cortisol levels confirm stress, and (iv) the subject confirmed with the stress. Next, a plot (chart) is drawn using the sensing values on time and sensor axis for the given episode.

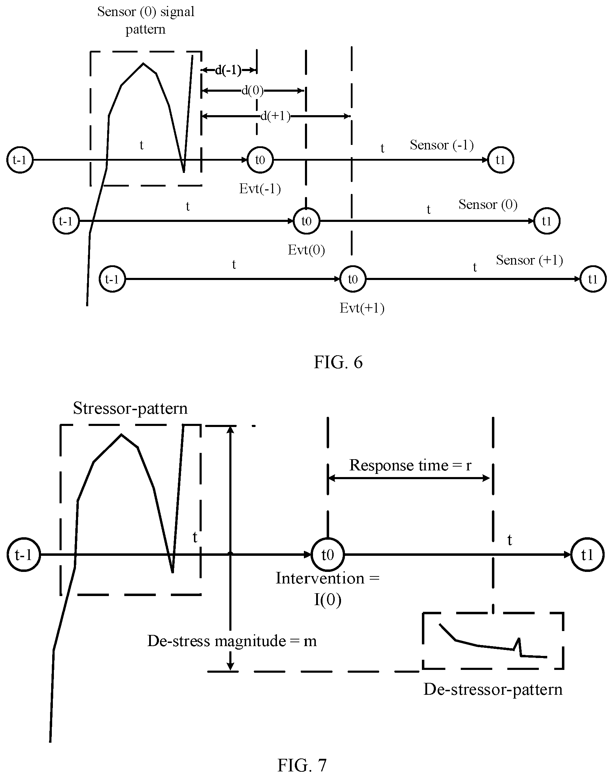

FIG. 6 is an exemplary snapshot of a chart showing stress sensor signal patterns, in accordance with some embodiments of the present disclosure. As shown in FIG. 6, the snapshot of this chart (literally an image) is then labelled as stress patterns, along with the stress labels (physical, mental, embarrassing, etc.). Lastly, these snapshots (images) and respective labels are then fed as input to the CNN model for training to obtain the pre-trained stressor estimation model.

An exemplary process of the multi-model dialogue is explained below through the pre-trained large language model (LLM) agent to estimate different stress patterns (events) and are used to estimate the stressors through the pre-trained stressor estimation model:

1. The pre-trained large language model (LLM) agent to formulate a message (the initial dialogue context) for conversation.

```
Example:
{
        "prompt" : "create a conversational message for enquiring if $subject felt
stressed based on following group of $reasons indicating unusual events.
Where $subject is the name of a person and $reasons is a json array object
containing all the reasons to believe person is stressed. Keep the message short
and ask if the person would like to discuss it. Respond in a json object with
$message being the key and message being the value".
        "reasons": [
        "detected uncategorized physical activity",
        "physical activity intensity higher than average",
        "physical activity intensity outside of standard deviations
        range",
        "physical activity episode lasted longer than usual",
        "physical intense activity episode in late evening",
        "at work location during unusual hours"
```

],

2. The following is an exemplary initial dialogue context from the pre-trained large language model (LLM) agent:

```
        "$message": "Hey $subject, just wanted to check in - noticed you
performed some unusually high intensity physical activity for a longer duration,
especially late in the evening yesterday at work. Everything okay on your end,
would you like to discuss?"
}
```

3. Similar messages (subsequent dialogue contexts) can be presented to the subject for interactive conversation along with an interface for the subject to respond.
Example:
Choose one:
A. Yes B. No

4. If the subject chooses "Yes" then the conversation is continued further and terminates the process on "No".

5. The subject's email and calendar is accessed, which it uses to proceed further in conversation.

6. All the calendar invites and direct emails to the subject are extracted, preceding "x" amount of time before the probable-stress-episode.

7. The pre-trained large language model (LLM) agent is employed to look for any direct-action items for subject in meeting agenda, meeting minutes and in direct emails. Example:

```
{
        "prompt": "$meeting_agendas is a json array object containing all the
meeting agendas, $meeting_minutes is a json array object containing all the
meeting minutes and likewise $email_messages is a json array object
containing all the email messages addressed directly to the subject in the $time
amount of time preceding the probable-stress-episode. Review the contents of
$meeting_agendas, $meeting_minutes, and $email_messages, and identify all
the immediate and direct action items addressed to the subject. Respond as a
json array of objects, where each object summarizes each action item and adds
it to $ summary, limit the size of each $summary to be short and precise, also
evaluate minimum of 3 and maximum of 5 emotions expressed into
$emotions.$expressed or evoked into $emotions.$evoked by the tone of the
input text from $meeting_minutes, $meeting_agenda, or $email_message."
{
"action_items": [
        {
        "$timestamp": "2024-09-08T18:15:41-12:00",
                        "$source": "email",
                        "$message": {
                        "$subject": "Urgent: Need Regional Analysis Report",
                        "$from": "sales.head@corporate.com",
                        "$content": "Required by tomorrow morning. I need the
                        regional analysis report for the quarter-end on my desk
                        by tomorrow morning without fail. This cannot be
                        delayed any further. Make sure it's complete and
                        thoroughly reviewed."
                        },
        "$summary": "Prepare the regional analysis reports for the
        quarter end for submission no later than September 9th",
                        "$emotions.$expressed": ["urgency", "frustration", "
                        impatience", "dissatisfaction"],
        "$emotions.$evokes": ["stress", "pressure", "anxiety", "
            fear"]
                        }
        ]
}
```

8. Once the summarized action_items are available, then the pre-trained large language model (LLM) agent is employed to iterate and structure the next conversational message for the subject.

```
Example:
{
"prompt": "There was an action item directly addressed to $subject, and
$summary contains the summary of what is expected from $subject, this
summary was extracted from $action_item[i].$source, sent by
$action_item[i].$message.$from and original message seems to express
$action_item[i].$emotions.$expressed and seems to evoke
```

```
$action_item[i].$emotions.$evokes emotions for $subject. Based on the above
context, compile a short comforting chat message also questioning if this was
the reason for $probable-stress-episodes.$reasons. And offer to help",
}
```

9. Exemplary subsequent message (subsequent dialogue context) from the pre-trained large language model (LLM) agent to the subject.

$message: "I saw the message from sales head about the quarter-end regional analysis reports due tomorrow morning. It seemed pretty urgent and intense - was that why you seemed busy working late yesterday? "

10. Allow the subject to respond to this message.

Example: "There were so many physical documents which I had to gather from different departments and review them to summarize the final report. And yes, it is stressful and embarrassing."

11. Extract further directions to take from the subject's message using the pre-trained large language model (LLM) agent.

```
{
"prompt": "Respond in a json object $response.$stressed as
YES/NO/UNANSWERED which is set to YES if the $message is affirmative
about being stressed, NO if it is not, and UNANSWERED if it is not answered.
Identify the kind of stress expressed by $subject in
$response.$self_declared_stress."
}
```

12. If the $response.$stressed is YES, then annotate the probable-stress-episode as a stress-episode. Create a snapshot of features involved in the episode as $identified-stress-patterns and export it as a csv and assign stress labels: Again, derived using the pre-trained large language model (LLM) agent based on $probable-stress-pattern.$reasons and $response.$self_declared_stress. While features involved in detecting stress patterns are recorded, but system also retains the other features during the entire episode as $other-feature-patterns. The labels in this example will be as follows:

```
{
"$identified-stress-patterns": "subject-20240908T2323411200.csv",
"$other-feature-patterns":"subject-other-features-
20240908T2323411200.csv",
        "$labels": [ "physical stress", "pressure", "anxiety", "embarrassment"],
}
```

13. This concludes the annotation part for stress patterns, these annotations are then used as inputs to the pre-trained stressor estimation model to estimate the stressors. Similar method is applied to identify and annotate the de-stressing patterns.

14. From the 9th to 12th step above the email sent by sales.head@corporate.com has been identified as the stressor and confirmed by the subject.

15. Now, more information about the identified stressor is learned by continuing the conversation with the subject.

16. All the emails from sales.head@corporate.com addressed directly to the subject in the last few months are identified.

```
{
"prompt": "From all emails in $all_emails_from_find_result filter only those
emails which have subject like $action_items[i]. $message.$subject and the
email content in $action_items[i].$message.$content expects some deliverables
from the subject and put it in $filtered_email_list. Check if these emails are sent
on any specific days of the month and specify this in terms of $frequency
DAILY/WEEKLY/MONTHLY/QUARTERLY; and find any commonalities
in the kind of deliverables these emails are expecting in $deliverables."
}
Resulting json object as below:
{
$filtered_email_list: [ an array of emails],
$frequency: "QUARTERLY",
$deliverables: [ "statements", "reports", "analysis"]
}
```

17. The subject is then asked to confirm if all other filtered emails are consistent with the frequency of occurrence and whether they caused similar stressful episodes for the subject. If the subject responds affirmatively, then the above object is recorded and labelled as a stressor and also establishes a link to the stress-pattern annotated above.

The the one or more stressors estimated at this step are to be relieved from the subject with suitable de-stressor mechanisms. Thus, each stressor type has a related de-stressor mechanism which is to be recommended to the subject to get rid of the stress. Hence, at step 310 of the method 300, the one or more hardware processors 104 of the system 100 are configured to recommend a de-stressor for the subject against each of the one or more stressors estimated at step 308 of the method 300. In an embodiment, the personalization module 206 is configured to recommend the personalized de-stress mechanism based on the one or more de-stressors identified by the de-stressor identification module 210 through the de-stress recommendation module 208.

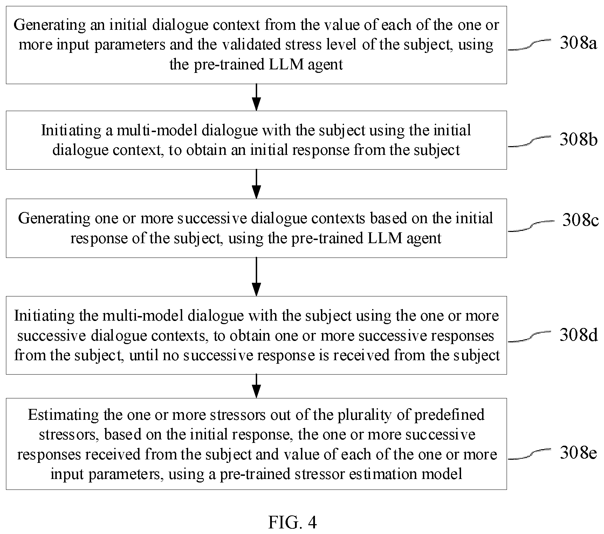

A pre-configured recommendation lookup table is employed to identify suitable de-stressor mechanism for each stressor estimated. FIG. 4 is a flowchart showing the steps for generating a pre-configured recommendation lookup table, in accordance with some embodiments of the present disclosure. As shown in FIG. 4, generating the pre-configured recommendation lookup table is explained through steps 310a to 310d. At step 310a, one or more historical knowledge documents and one or more literature surveys, are received from a knowledge repository. The one or more historical knowledge documents and the one or more literature surveys are the knowledge available in the art and curated using techniques such as natural language processing (NLP).

At step 310b, a plurality of stressors and a plurality of de-stressors are identified from the one or more historical knowledge documents and the one or more literature surveys received at step 310a. An information retrieval technique such as a Term Frequency and Inverse Document Frequency (TF/IDF) technique is employed to identify the plurality of stressors and the corresponding plurality of de-stressors. The plurality of stressors is the list of stressor types which are used as the plurality of predefined stressors at step 308 of the method 300. Similarly, the plurality of de-stressors is the list of de-stressor types for the identified list of plurality of stressors. It is possible that each stressor type may have correspondence with more than one de-stressor type, or vice versa based on their inter-dependencies. The plurality of stressors and the plurality of de-stressors are represented in the form of curated lookup tables.

At step 310c, a recommendation knowledge graph is generated using the plurality of stressors and the plurality of de-stressors identified at step 310b. In the present disclosure, a Markov's Decision Process (MDP) is employed where a reinforcement learning (RL) agent generates the relationship between the plurality of stressors and the plurality of de-stressors using the reward function. The recommendation knowledge graph comprises the plurality of stressors and the plurality of de-stressors as nodes and their relationships are represented using the respective edges (between the nodes). The goal of the RL agent is to traverse from the stressor node to the intervention node (de-stressor node) with maximum reward accumulation.

1. There two kinds of nodes in this graph:

    a. stressor-node: starting nodes for traversal.
    b. de-stressor-node: Can also be called intervention-node, or the goal-nodes for traversal.

2. The initial weightages for rewards are arrived at by computing from the curated lookup tables. These initial weightages change as the subject starts using the system, and the system re-evaluates the weightage based on automated model.
3. The graph links with maximum reward (100) are created using the curated lookup table mentioned above.
4. There may exist stressors which may not have direct interventions listed in the curated lookup table. Such stressor nodes are linked according to stressor grouping. The stressor-grouping is created based on factors:

    a. The curated by the subject-matter-experts using the literature survey and their practicing knowledge.
    b. Or based on common attributes those which are derived based on sensing outcomes. Example: All stressors identified at a particular location can form a group and can be linked to each other using minimum reward (0).

5. Similarly, the intervention can also be grouped.
6. Using the Q-Learning algorithm, an initial recommendation Q-Table is computed as follows:

$$Q(stressor, \ de-stressor)$$
$$\leftarrow R(stressor, \ de-stressor) \ + \ \gamma$$
$$\cdot Max[Q(next, \ all-de-stressor)]$$

7. The Q-Table is a matrix of stressor vs de-stressors with traversal rewards. An exemplary Q-Table is shown in below Table 2:

Table 2

|  | A | K | B | S |  | H | V | P | D | G |
|---|---|---|---|---|---|---|---|---|---|---|
| 06 | -1 | -1 | 100 | -1 |  | -1 | -1 | -1 | -1 | -1 |
| : |  |  |  |  |  |  |  |  |  |  |
| 24 | 0 | 100 | -1 | -1 |  | -1 | -1 | -1 | -1 | -1 |
| : |  |  |  |  |  |  |  |  |  |  |
| 22 | 100 | 100 | -1 | -1 |  | -1 | -1 | -1 | -1 | -1 |

8. FIG. 7 is an exemplary chart for calculating weightages for de-stressor, in accordance with some embodiments of the present disclosure. As shown in FIG. 7, these weightages are a function of response-time (r) since the de-stressor (intervention) and magnitude (m) of change in the stress pattern. The combination of shorter response-time and larger magnitude of change is considered better; hence, the function must result in higher weightage.

Thus, weightage (w) for intervention node I(0) is computed as:

$$w_{I(0)} = \frac{m}{r}$$

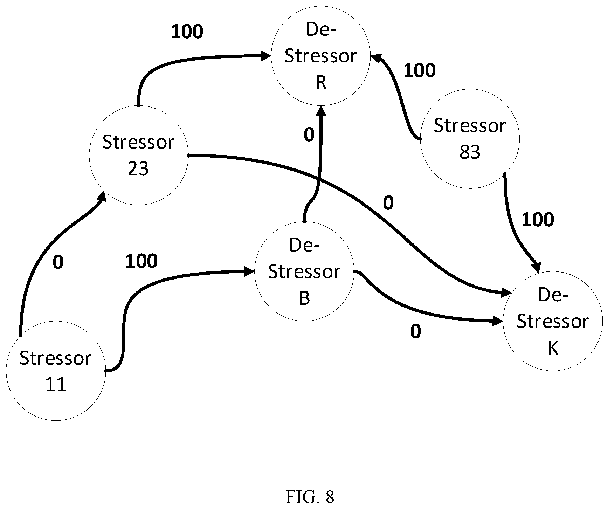

9. The recommendation knowledge graph is generated using the plurality of stressors and the plurality of de-stressors with the help of the Q-table. FIG. 8 shows an exemplary recommendation knowledge graph, in accordance with some embodiments of the present disclosure.

At step 310d, the pre-configured recommendation lookup table is generated from the recommendation knowledge graph using a graph traversal technique. The graph traversal technique is employed to identify one or more de-stressors (de-stressor nodes) for each given stressor (stressor nodes).

The recommendation knowledge graph is employed to recommend one or more de-stressors using the graph traversal technique and the reinforcement learning (RL) agent. Table 3 shows an exemplary stressor and de-stressor relationship table:

Table 3

| De-stressor | De-stressor-pattern | Stressor | Magnitude (change in cortisol value) M | Duration from Stress to De-stress R | M:R (Ratio) |
|---|---|---|---|---|---|
| Music | Activity indicators, cortisol value indicators | Deliverables | Cv | Slot-group | X |

The methods and systems of the present disclosure automatically detect the stress of the subject using the input parameters of the subject, estimate the stressors, and provide the personalized de-stressor recommendation based on stressor estimation. Thus, the methods and systems of the present disclosure for the stress detection and personalized de-stressor recommendation is simple, convenient and effective. The stress of the subject is detected using a two-level validation mechanism where in the first level, the stress is detected using the input parameters such as the one or more physiological parameters, the one or more psychological parameters, the one or more behavioral parameters, and the one or more environmental parameters, and in the second level, the detected stress in the first level is validated using the bio-chemical indicator such as the cortisol level. Thus, the present disclosure detects the stress of the subject most accurately and accordingly the de-stressors are recommended for the speedy recovery from the stress.

The methods and systems of the present disclosure can be effectively implemented for the subjects working in corporate environments especially in IT workplaces where the work stress with time deadlines is most common situation. However, the scope is not limited to other applications such as rehabilitation centers, family counselling, and other related applications where the stress is most prominent.

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

The embodiments of present disclosure herein address unresolved problems of automated personalized de-stressor recommendation based on stressor estimation. According to the present disclosure, the methods and systems begin by detecting the stress using multi-model evaluation design including physiological changes, psychological changes, behavioral changes, environmental changes, and a bio-chemical indicator. Next, the stressors related to the stress detection are identified using a large language model (LLM), which correlates with stress patterns to create a personalized model. Then, appropriate de-stressors are recommended using a recommendation lookup table.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means, and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

## Claims

1. A processor-implemented method (300), comprising:

   receiving, via one or more input/output (I/O) interfaces, a value of each of one or more input parameters of a subject whose stressor is to be estimated, and a personalized de-stressor is to be recommended, wherein the one or more input parameters of the subject comprises one or more physiological parameters, one or more psychological parameters, one or more behavioral parameters, one or more environmental parameters, and a bio-chemical indicator (302);
   determining, via one or more hardware processors, a stress level of the subject based on the value of each of the one or more input parameters using a predefined stress detection criterion, wherein the predefined stress detection criterion is defined with one or more boundary conditions associated with the one or more input parameters (304);

validating, via the one or more hardware processors, the stress level of the subject using the value of the bio-chemical indicator and a ground truth bio-chemical indicator value, to obtain a validated stress level of the subject (306);

estimating, via the one or more hardware processors, one or more stressors out of a plurality of predefined stressors, based on the validated stress level of the subject, using a pre-trained large language model (LLM) agent (308); and

recommending, via the one or more hardware processors, a de-stressor for the subject against each of the one or more stressors estimated, using a pre-configured recommendation lookup table (310).

2. The processor-implemented method (300) as claimed in claim 1, wherein:

the one or more physiological parameters comprises a body temperature, a blood pressure, a blood sugar level, a fatigue tracking, a cardiac screening, and an activity tracking,

the one or more psychological parameters comprises an emotion level, and an anxiety level,

the one or more behavioral parameters comprises a sleep deprivation, an alcohol abuse detection, a smoking detection, and an activity pattern detection, and

the one or more environmental parameters comprises one or more location related parameters of one or more environments, one or more temporal related parameters, and one or more digital related parameters, associated with the subject.

3. The processor-implemented method (300) as claimed in claim 1, wherein estimating the one or more stressors out of the plurality of predefined stressors, based on the validated stress level of the subject, using the pre-trained large language model (LLM) agent, comprises:

generating an initial dialogue context from the value of each of the one or more input parameters and the validated stress level of the subject, using the pre-trained LLM agent (308a);

initiating a multi-model dialogue with the subject using the initial dialogue context, to obtain an initial response from the subject (308b);

generating one or more successive dialogue contexts based on the initial response of the subject, using the pre-trained LLM agent (308c);

initiating the multi-model dialogue with the subject using the one or more successive dialogue contexts, to obtain one or more successive responses from the subject, until no successive response is received from the subject (308d); and

estimating the one or more stressors out of the plurality of predefined stressors, based on the initial response, the one or more successive responses received from the subject and value of each of the one or more input parameters, using a pre-trained stressor estimation model (308e).

4. The processor-implemented method (300) as claimed in claim 1, wherein the pre-configured recommendation lookup table is generated by:

receiving one or more historical knowledge documents and one or more literature surveys, from a knowledge repository (310a);

identifying a plurality of stressors and a plurality of de-stressors, from the one or more historical knowledge documents and the one or more literature surveys using an information retrieval technique (310b);

creating a recommendation knowledge graph using the plurality of stressors and the plurality of de-stressors, using a Markov's Decision Process (MDP) (310c); and

generating the pre-configured recommendation lookup table from the recommendation knowledge graph using a graph traversal technique (310d).

5. The processor-implemented method (300) as claimed in claim 1, wherein the bio-chemical indicator is a cortisol level.

6. A system (100), comprising:

a memory (102) storing instructions;

one or more input/output (I/O) interfaces (106);

one or more hardware processors (104) coupled to the memory (102) via the one or more I/O interfaces (106), wherein the one or more hardware processors (104) are configured by the instructions to:

receive via the one or more input/output (I/O) interfaces (106), a value of each of one or more input parameters of a subject whose stressor is to be estimated, and a personalized de-stressor is to be recommended, wherein the one or more input parameters of the subject comprises one or more physiological parameters, one or more psychological parameters, one or more behavioral parameters, one or more environmental parameters, and a bio-chemical indicator;

determine a stress level of the subject based on the value of each of the one or more input parameters using a predefined stress detection criterion, wherein the predefined stress detection criterion is defined with one or more boundary conditions associated with the one or more input parameters;

validate the stress level of the subject using the value of the bio-chemical indicator and a ground truth bio-chemical indicator value, to obtain a validated stress level of the subject;

estimate one or more stressors out of a plurality of predefined stressors, based on the validated stress level of the subject, using a pre-trained large language model (LLM) agent; and

recommend a de-stressor for the subject against each of the one or more stressors estimated, using a pre-configured recommendation lookup table.

7. The system (100) as claimed in claim 6, wherein:

the one or more physiological parameters comprises a body temperature, a blood pressure, a blood sugar level, a fatigue tracking, a cardiac screening, and an activity tracking,

the one or more psychological parameters comprises an emotion level, and an anxiety level,

the one or more behavioral parameters comprises a sleep deprivation, an alcohol abuse detection, a smoking detection, and an activity pattern detection, and

the one or more environmental parameters comprises one or more location related parameters of one or more environments, one or more temporal related parameters, and one or more digital related parameters, associated with the subject.

8. The system (100) as claimed in claim 6, wherein the one or more hardware processors (104) are configured to estimate the one or more stressors out of the plurality of predefined stressors, based on the validated stress level of the subject, using the pre-trained large language model (LLM) agent, by:

generating an initial dialogue context from the value of each of the one or more input parameters and the validated stress level of the subject, using the pre-trained LLM agent;

initiating a multi-model dialogue with the subject using the initial dialogue context, to obtain an initial response from the subject;

generating one or more successive dialogue contexts based on the initial response of the subject, using the pre-trained LLM agent;

initiating the multi-model dialogue with the subject using the one or more successive dialogue contexts, to obtain one or more successive responses from the subject, until no successive response is received from the subject; and

estimating the one or more stressors out of the plurality of predefined stressors, based on the initial response, the one or more successive responses received from the subject and value of each of the one or more input parameters, using a pre-trained stressor estimation model.

9. The system (100) as claimed in claim 6, wherein the one or more hardware processors (104) are configured to generate the pre-configured recommendation lookup table, by:

receiving one or more historical knowledge documents and one or more literature surveys, from a knowledge repository;

identifying a plurality of stressors and a plurality of de-stressors, from the one or more historical knowledge documents and the one or more literature surveys using an information retrieval technique;

creating a recommendation knowledge graph using the plurality of stressors and the plurality of de-stressors, using a Markov's Decision Process (MDP); and

generating the pre-configured recommendation lookup table from the recommendation knowledge graph using a graph traversal technique.

10. The system (100) as claimed in claim 6, wherein the bio-chemical indicator is a cortisol level.

11. One or more non-transitory machine-readable information storage mediums comprising one or more instructions which when executed by one or more hardware processors cause:

receiving a value of each of one or more input parameters of a subject whose stressor is to be estimated, and a personalized de-stressor is to be recommended, wherein the one or more input parameters of the subject comprises one or more physiological parameters, one or more psychological parameters, one or more behavioral parameters, one or more environmental parameters, and a bio-chemical indicator;

determining a stress level of the subject based on the value of each of the one or more input parameters using a predefined stress detection criterion, wherein the predefined stress detection criterion is defined with one or more boundary conditions associated with the one or more input parameters;

validating the stress level of the subject using the value of the bio-chemical indicator and a ground truth bio-chemical indicator value, to obtain a validated stress level of the subject;

estimating one or more stressors out of a plurality of predefined stressors, based on the validated stress level of the subject, using a pre-trained large language model (LLM) agent; and

recommending a de-stressor for the subject against each of the one or more stressors estimated, using a pre-configured recommendation lookup table.

12. The one or more non-transitory machine-readable information storage mediums as claimed in claim 11, wherein:

the one or more physiological parameters comprises a body temperature, a blood pressure, a blood sugar level, a fatigue tracking, a cardiac screening, and an activity tracking,

the one or more psychological parameters comprises an emotion level, and an anxiety level,

the one or more behavioral parameters comprises a sleep deprivation, an alcohol abuse detection, a smoking detection, and an activity pattern detection, and

the one or more environmental parameters comprises one or more location related parameters of one or more environments, one or more temporal related parameters, and one or more digital related parameters, associated with the subject.

13. The one or more non-transitory machine-readable information storage mediums as claimed in claim 11, wherein estimating the one or more stressors out of the plurality of predefined stressors, based on the validated stress level of the subject, using the pre-trained large language model (LLM) agent, comprises:

generating an initial dialogue context from the value of each of the one or more input parameters and the validated stress level of the subject, using the pre-trained LLM agent;

initiating a multi-model dialogue with the subject using the initial dialogue context, to obtain an initial response from the subject;

generating one or more successive dialogue contexts based on the initial response of the subject, using the pre-trained LLM agent;

initiating the multi-model dialogue with the subject using the one or more successive dialogue contexts, to obtain one or more successive responses from the subject, until no successive response is received from the subject; and

estimating the one or more stressors out of the plurality of predefined stressors, based on the initial response, the one or more successive responses received from the subject and value of each of the one or more input parameters, using a pre-trained stressor estimation model.

14. The one or more non-transitory machine-readable information storage mediums as claimed in claim 11, wherein the pre-configured recommendation lookup table is generated by:

receiving one or more historical knowledge documents and one or more literature surveys, from a knowledge repository;

identifying a plurality of stressors and a plurality of de-stressors, from the one or more historical knowledge documents and the one or more literature surveys using an information retrieval technique;

creating a recommendation knowledge graph using the plurality of stressors and the plurality of de-stressors, using a Markov's Decision Process (MDP); and

generating the pre-configured recommendation lookup table from the recommendation knowledge graph using a graph traversal technique.

15. The one or more non-transitory machine-readable information storage mediums as claimed in claim 11, wherein the bio-chemical indicator is a cortisol level.

System **100**

**108**

Memory **102**

Modules **102a**

Repository **102b**

I/O interface(s) **106**

Hardware processor(s) **104**

FIG. 1

FIG. 2

300

Receive a value of each of one or more input parameters of a subject whose stressor is to be estimated, and a personalized de-stressor is to be recommended, wherein the one or more input parameters of the subject comprises (i) one or more physiological parameters, one or more psychological parameters, one or more behavioral parameters, one or more environmental parameters, and a bio-chemical indicator ⟋ 302

Determine a stress level of the subject based on the value of each of the one or more input parameters using a predefined stress detection criterion, wherein the predefined stress detection criterion is defined with one or more boundary conditions associated with the one or more input parameters ⟋ 304

Validate the stress level of the subject using the value of the bio-chemical indicator and a ground truth bio-chemical indicator value, to obtain a validated stress level of the subject ⟋ 306

Estimate one or more stressors out of a plurality of predefined stressors, based on the validated stress level of the subject, using a pre-trained large language model (LLM) agent ⟋ 308

Recommend a de-stressor for the subject against each of the one or more stressors estimated, using a pre-configured recommendation lookup table ⟋ 310

FIG. 3

Generating an initial dialogue context from the value of each of the one or more input parameters and the validated stress level of the subject, using the pre-trained LLM agent
308a

Initiating a multi-model dialogue with the subject using the initial dialogue context, to obtain an initial response from the subject
308b

Generating one or more successive dialogue contexts based on the initial response of the subject, using the pre-trained LLM agent
308c

Initiating the multi-model dialogue with the subject using the one or more successive dialogue contexts, to obtain one or more successive responses from the subject, until no successive response is received from the subject
308d

Estimating the one or more stressors out of the plurality of predefined stressors, based on the initial response, the one or more successive responses received from the subject and value of each of the one or more input parameters, using a pre-trained stressor estimation model
308e

FIG. 4

| Receiving one or more historical knowledge documents and one or more literature surveys, from a knowledge repository | 310a |

| Identifying a plurality of stressors and a plurality of de-stressors, from the one or more historical knowledge documents and the one or more literature surveys using an information retrieval technique | 310b |

| Creating a recommendation knowledge graph using the plurality of stressors and the plurality of de-stressors, using a Markov's Decision Process (MDP) | 310c |

| Generating the pre-configured recommendation lookup table from the recommendation knowledge graph using a graph traversal technique | 310d |

FIG. 5

FIG. 6

FIG. 7

FIG. 8

Europäisches
Patentamt

European
Patent Office

Office européen
des brevets

# EUROPEAN SEARCH REPORT

**Application Number**

EP 25 20 2462

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2017/251967 A1 (PREMSUKH HARDY [CA]) 7 September 2017 (2017-09-07) | 1-3,5-8, 10-13,15 | INV. G16H10/20 |
| A | * The whole document, in particular: Paragraphs [0057], [0058], [0060], [0061], [0063], [0064], [0066], [0072], [0085], [0086]; Figures 3, 9b; Claims 1, 8, 14. * | 4,9,14 | A61B5/16 G06F18/00 G06N3/02 G06N5/00 G06N7/01 |
| | ----- | | |
| Y | DONGRE POORVESH: "Physiology-Driven Empathic Large Language Models (EmLLMs) for Mental Health Support", COMPANION PROCEEDINGS OF THE 29TH INTERNATIONAL CONFERENCE ON INTELLIGENT USER INTERFACES, ACMPUB27, NEW YORK, NY, USA, 11 May 2024 (2024-05-11), pages 1-5, XP059702718, DOI: 10.1145/3613905.3651132 ISBN: 979-8-4007-0511-3 | 1-3,5-8, 10-13,15 | G16H20/70 G16H40/60 G16H50/70 |
| A | * The whole document, in particular: Pages 1 - 4; Figure 1. * | 4,9,14 | |
| | ----- | | **TECHNICAL FIELDS SEARCHED (IPC)** |
| Y | FANG CATHY MENGYING ET AL: "PhysioLLM: Supporting Personalized Health Insights with Wearables and Large Language Models", 2024 IEEE EMBS INTERNATIONAL CONFERENCE ON BIOMEDICAL AND HEALTH INFORMATICS (BHI), IEEE, 10 November 2024 (2024-11-10), pages 1-8, XP034863651, DOI: 10.1109/BHI62660.2024.10913781 [retrieved on 2025-03-17] | 1-3,5-8, 10-13,15 | G16H G06F G06E A61B G06N |
| A | * The whole document, in particular: Pages 1 - 7; Figure 1. * | 4,9,14 | |
| | ----- | | |
| | -/-- | | |

The present search report has been drawn up for all claims

| Place of search | Date of completion of the search | Examiner |
|---|---|---|
| Munich | 11 February 2026 | Ruschke, Stefan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................

& : member of the same patent family, corresponding document

EPO FORM 1503 03.82 (P04C01)

**page 1 of 2**

Europäisches
Patentamt

European
Patent Office

Office européen
des brevets

# EUROPEAN SEARCH REPORT

**Application Number**

**EP 25 20 2462**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,P | THAPA BISHAL ET AL: "StressLLM: Large Language Models for Stress Prediction via Wearable Sensor Data", 2025 IEEE INTERNATIONAL CONFERENCE ON CONSUMER ELECTRONICS (ICCE), IEEE, 26 March 2025 (2025-03-26), pages 1-6, XP034872925, DOI: 10.1109/ICCE63647.2025.10929774 [retrieved on 2025-03-26] * The whole document. * ----- | 1-15 | |

| | | | TECHNICAL FIELDS SEARCHED (IPC) |
|---|---|---|---|

The present search report has been drawn up for all claims

| Place of search | Date of completion of the search | Examiner |
|---|---|---|
| Munich | 11 February 2026 | Ruschke, Stefan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.............................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 20 2462

This annex lists the patent family members relating to the patent documents cited in the above-mentioned European search report.
The members are as contained in the European Patent Office EDP file on
The European Patent Office is in no way liable for these particulars which are merely given for the purpose of information.

11-02-2026

| Patent document cited in search report | Publication date | Patent family member(s) | | Publication date |
|---|---|---|---|---|
| US 2017251967 A1 | 07-09-2017 | US | 2017251967 A1 | 07-09-2017 |
| | | WO | 2017147715 A1 | 08-09-2017 |

EPO FORM P0459

For more details about this annex : see Official Journal of the European Patent Office, No. 12/82

29

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IN 202421093803